# EUROPEAN PATENT APPLICATION

(11) **EP 4 582 748 A1**
(43) Date of publication of application: **09.07.2025**
(21) Application number: 23858643.2
(22) Date of filing: 31.03.2023
(51) Int. Cl.: F24F 8/30, F24F 8/192, F24F 8/20, F24F 13/20, F24F 13/00, F24F 1/0071

(54) **AIR PURIFICATION DEVICE AND AIR CONDITIONER**

(30) Priority: 31.08.2022 CN 202211065958; 31.08.2022 CN 202222329915 U
(71) Applicant: GD Midea Air-Conditioning Equipment Co., Ltd., Foshan, Guangdong 528311 (CN)
(72) Inventor: DAI, Guangjian, Foshan, Guangdong 528311 (CN)
(74) Representative: RGTH
(86) International application number: PCT/CN2023/085573
(87) International publication number: WO 2024/045602

(57) **Abstract**

An air purification device and an air conditioner, the air purification device (1000) comprising a housing (100) and an ion generation assembly (200). The housing (100) comprises a protective cover (110) and a base (120) connected to each other. The ion generation assembly (200) comprises at least one first electrode (210), a second electrode (220) and a third electrode (230), the first electrode (210) being used for discharging to generate negative ions, and the second electrode (220) and the third electrode (230) being spaced apart and used for generating plasma cooperatively. The protective cover (110) is of a hollowed-out structure, the hollowed-out structure covering the ion generation assembly (200). The first electrode (210) and the second electrode (220) are connected to the base (120), and the third electrode (230) is connected to the inner side of the protective cover (200).

## Description

### CROSS-REFERENCE TO RELATED APPLICATIONS

This application claims priority to Chinese Patent Application No. 202211065958.X filed August 31, 2022 and entitled "AIR PURIFICATION DEVICE AND AIR CONDITIONER", and Chinese Patent Application No. 202222329915.X filed August 31, 2022 and entitled "AIR PURIFICATION DEVICE AND AIR CONDITIONER", the entire contents of each of which are incorporated herein by reference for all purpose.

### FIELD

The present disclosure relates to the field of air-conditioning equipment, and in particular, to an air purification device and an air conditioner.

### BACKGROUND

Both negative ion generators and plasma generators have a function of purifying air, and each has its own advantages. In the related art, a negative ion generator and a plasma generator are combined together for purification, but the structural design is unreasonable and thus it is difficult to balance the functions of negative ions and plasma, resulting in ineffective purification.

### SUMMARY

The present disclosure aims to at least partially resolve one of the problems in the related art. To this end, the present disclosure provides an air purification device, which can generate negative ions and plasmas to purify air. The structural design is more reasonable with higher compatibility, and effectively improves the purification effect.

In accordance with a first aspect of the present disclosure, an embodiment provides an air purification device, comprising: a housing, comprising a protective cover and a base connected to the protective cover;
an ion generation assembly, comprising at least one first electrode, a second electrode, and a third electrode, where the at least one first electrode is configured for discharging to generate negative ions, and the second electrode is spaced apart from the third electrode and is configured for cooperating with the third electrode to generate plasmas;
where the protective cover is of a hollowed-out structure, the hollowed-out structure covers the ion generation assembly, the at least one first electrode and the second electrode are fixedly connected to the base, and the third electrode is fixedly connected to an inner side of the protective cover.

The air purification device according to the embodiments of the present disclosure at least has the following beneficial effects.

The ion generation assembly uses the high-voltage discharge of the at least one first electrode to generate negative ions, and the second electrode and the third electrode are respectively arranged on the base and the protective cover, such that the second electrode and the third electrode are spaced apart to form a strong electric field, to generate plasmas. The generated negative ions and plasmas can diffuse into the air through the hollowed-out structure to sterilize the air, can kill bacteria and viruses in the air entering the protective cover, and also provides a certain odor removal effect. The air purification device of the present disclosure has the advantages of purification using negative ions and purification using plasmas and effectively improves the purification effect. The protective cover can effectively protect the electrodes therein, and can also prevent a human body from accessing the electrodes in the protective cover, thereby improving the usage safety.

According to some embodiments of the present disclosure, the at least one first electrode and the second electrode are electrically connected, and are connected to a high-voltage electrode of a power supply through a first conductive member, and the third electrode is connected to a ground electrode of the power supply through a second conductive member.

According to some embodiments of the present disclosure, the at least one first electrode and the second electrode are fixedly connected to form a unity or are connected by a wire.

According to some embodiments of the present disclosure, the at least one first electrode and the second electrode extend into the base, and the first conductive member is connected to the at least one first electrode or the second electrode in the base and extends out from the base to connect to the high-voltage electrode of the power supply.

According to some embodiments of the present disclosure, the second conductive member is connected to the third electrode in the protective cover and extends toward the base, and the second conductive member extends into the base and extends out from the base to connect to the ground electrode of the power supply.

According to some embodiments of the present disclosure, the second electrode comprises an electrode plate. The electrode plate comprises a plurality of second tip portions arranged in a length direction of the electrode plate, and the at least one first electrode is connected to an end of the electrode plate.

According to some embodiments of the present disclosure, at least two first electrodes are provided, and the at least two first electrodes are divided into two groups and respectively arranged at two ends of the electrode plate, or the at least two first electrodes are both arranged at one end of the electrode plate.

According to some embodiments of the present disclosure, the second tip portion is of a tooth-like, needle-tip, or filament-like structure.

According to some embodiments of the present disclosure, a connecting rib is arranged on a side wall of the protective cover away from the base, the third electrode is arranged on a side of the connecting rib facing the base, and the connecting rib, the second electrode, and the third electrode respectively extend in a length direction of the base.

According to some embodiments of the present disclosure, a length by which the third electrode extends in the length direction of the base is less than or equal to a length of the connecting rib, and a width of the third electrode is less than or equal to a width of the connecting rib.

According to some embodiments of the present disclosure, shielding portions are arranged on the side wall of the protective cover away from the base, the shielding portions are respectively located at two ends of the connecting rib. Connecting portions are respectively arranged at two ends of the third electrode, and the connecting portions are connected to a side of the shielding portions facing the base.

According to some embodiments of the present disclosure, a length of the third electrode is greater than a length of a discharge portion of the second electrode.

According to some embodiments of the present disclosure, the third electrode and the second electrode are staggered in a height direction of the base.

According to some embodiments of the present disclosure, the protective cover comprises a frame and a plurality of grid bars connected to the frame. The plurality of grid bars are arranged at an interval in a length direction of the base to form a grille arrangement to construct the hollowed-out structure. A spacing is formed between adjacent grid bars. And a discharge portion of the at least one first electrode and/or the discharge portion of the second electrode is exposed through the spacing in the width direction of the base.

According to some embodiments of the present disclosure, the spacing between adjacent grid bars is less than or equal to 6 mm.

According to some embodiments of the present disclosure, the at least one first electrode comprises a basal portion and a first tip portion connected to a top end of the basal portion, and a height of the first tip portion is greater than a height of the basal portion.

According to some embodiments of the present disclosure, the first tip portion comprises a plurality of conductive fibers connected to the basal portion.

According to some embodiments of the present disclosure, a distance between the at least one first electrode and the third electrode is greater than a distance between the second electrode and the third electrode.

According to some embodiments of the present disclosure, a distance between the at least one first electrode and the protective cover is greater than or equal to 3 mm, and a distance between the second electrode and the protective cover is greater than or equal to 3 mm.

In accordance with a second aspect of the present disclosure, an embodiment provides an air conditioner, comprising the air purification device according to the embodiments of the first aspect.

Because the air conditioner adopts all the schemes of the air purification device of the above embodiments, the air conditioner has at least all the beneficial effects achieved by the schemes of the above embodiments.

Additional features and advantages of the disclosure will be set forth in the description which follows, and in part will be obvious from the description, or may be learned by the practice of the disclosure.

### BRIEF DESCRIPTION OF DRAWINGS

FIG. 1 is a schematic perspective diagram of an air purification device according to an embodiment of the present disclosure;
FIG. 2 is a schematic perspective diagram of a first electrodes and a second electrode in a connected configuration according to an embodiment of the present disclosure;
FIG. 3 is a schematic front view of a first electrodes and a second electrode in a connected configuration according to an embodiment of the present disclosure;
FIG. 4 is a schematic front view of an air purification device according to an embodiment of the present disclosure;
FIG. 5 is a schematic top view of an air purification device according to an embodiment of the present disclosure; and
FIG. 6 is a schematic side view of an air purification device according to an embodiment of the present disclosure.

List of reference numerals:
housing 100; negative ion generating area 101; plasma generating area 102; protective cover 110; grid bar 111; frame 112; connecting rib 113; shielding portion 114; base 120; first buckle 121; second buckle 122;
ion generation assembly 200; first conductive member 201; second conductive member 202; first electrode 210; first tip portion 211; basal portion 212; second electrode 220; electrode plate 221; second tip portion 222; third electrode 230; negative ion electrode 240; plasma high-voltage electrode 250; plasma ground electrode 260; and
air purification device 1000.

### DETAILED DESCRIPTION OF EMBODIMENTS

Embodiments of the present disclosure will be described in detail hereinafter with reference to accompanying drawings in which the same or like reference numerals refer to the same or like elements or elements having the same or like functions throughout. The embodiments described below with reference to the accompanying drawings are illustrative and are intended for illustration only and are not to be construed as limiting the present disclosure.

In the description of the present disclosure, it should be understood that for the description of orientations, the orientation or positional relationships indicated by the terms such as "on/upper" and "below/lower" are based on orientation or positional relationships shown in the accompanying drawings, and are used only for ease and brevity of illustration and description, rather than indicating or implying that the mentioned apparatus or element must have a particular orientation or must be constructed and operated in a particular orientation. Therefore, such terms should not be construed as limiting the present disclosure.

In the description of the present disclosure, the terms such as "first", "second" and the like used herein are merely used for distinguishing technical features, and are not intended to indicate or imply relative importance, or implicitly point out the number of the indicated technical features, or implicitly point out the order of the indicated technical features.

In the description of the present disclosure, unless otherwise explicitly defined, the terms such as "configure", "install/mount" and "connect" should be understood in a broad sense, and those having ordinary skills in the art can reasonably determine the specific meanings of the above terms in the present disclosure based on the specific contents of the scheme.

The schemes in the present disclosure will be described clearly and fully in conjunction with the accompanying drawings. Apparently, the embodiments described are merely some embodiments, rather than all of the embodiments of the present disclosure.

At present, a device that provides an air purification function by ionizing air mainly comprises a negative ion generator or a plasma generator. The negative ion generator can generate a large number of negative ions to combine with positively charged particles such as bacteria, viruses, and dust in the air, causing the particles to condense and settle, thereby reducing fine particles such as bacteria, viruses, and dust in the air. However, negative ions cannot really kill bacteria and viruses, and are not able to remove odors. The plasma generator can produce a large number of high-energy particles such as electrons and hydroxyl radicals, which can not only kill bacteria and viruses passing through the plasma area, but also decompose and purify peculiar odors in the air. However, the high-energy particles have high activity and short life, only exist in the tiny plasma area near the electrode, and cannot actively diffuse into the air to achieve an active sterilization effect, resulting in a limited range of function. It can be understood that air purification using negative ions or air purification using plasmas has its own advantages and disadvantages.

An air purification device 1000 provided by an embodiment of the present disclosure is suitable for use in an air conditioner, has the functions of a negative ion generator and a plasma generator, and therefore has the advantages of negative ions and plasmas. The air purification device 1000 can actively diffuse negative ions into the air to achieve a sterilization effect, can kill bacteria and viruses in the air passing through the air purification device 1000, and also provides a certain odor removal effect, such that indoor air can be purified to improve indoor air quality.

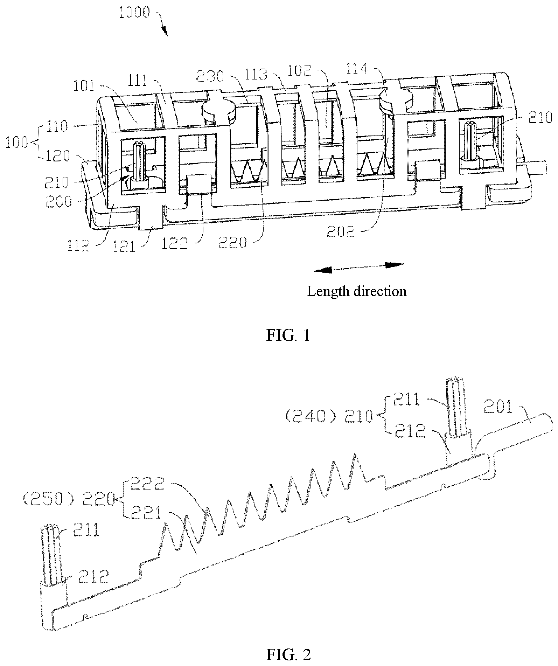

Referring to FIG. 1, the air purification device 1000 according to the embodiment of the present disclosure comprises a housing 100 and an ion generation assembly 200. The housing 100 comprises a protective cover 110 and a base 120. The protective cover 110 is connected to the base 120, such that a chamber is formed by the protective cover 110 and the base 120. The ion generation assembly 200 is mounted inside the chamber. The ion generation assembly 200 comprises first electrodes 210, a second electrode 220, and a third electrode 230. The first electrodes 210 are negative ion electrodes 240 configured for generating negative ions by high-voltage discharge. The second electrode 220 is spaced apart from the third electrode 230. The second electrode 220 and the third electrode 230 are respectively connected to electrodes of a power supply, such that there is a voltage difference between the second electrode 220 and the third electrode 230 to form a strong electric field, to generate plasmas. As such, the negative ions and the plasmas can be generated simultaneously, and air can be purified by the negative ions and the plasmas.

Referring to FIG. 1, it can be understood that the protective cover 110 is of a hollowed-out structure. The hollowed-out structure may be arranged as a grille,, a screen, or the like.

The hollowed-out structure is formed with a plurality of through-holes. The hollowed-out structure covers the ion generation assembly 200, and the negative ions generated can diffuse outward through the through-holes, to reduce fine particles such as bacteria, viruses, and dust in the air. In addition, air may also enter the chamber through the grille, and the plasmas kill bacteria and viruses in the air, and provides an odor removal effect. Therefore, the air purification device 1000 can take into account the advantages of negative ions purification and plasmas purification and provides a better purification effect. It should be noted that the air purification device 1000 may be mounted at an air outlet of an indoor unit of an air conditioner (not shown), an air flow generated at the air outlet during operation of the indoor unit of the air conditioner facilitates speeding up the diffusion of negative ions, and the air flowing through the air purification device 1000 is sterilized and deodorized by the plasmas. As such, indoor air can be effectively purified and the usage experience of the air conditioner can be improved. In some other embodiments of the present disclosure, the air purification device 1000 may be mounted at an air inlet of or in an air duct of the indoor unit of the air conditioner.

Referring to FIG. 1, it can be understood that the first electrodes 210 and the second electrode 220 are connected to the base 120, for example, fixedly or detachably, and the third electrode 230 is located above the second electrode 220 and is connected to an inner side of the protective cover 110, for example, fixedly or detachably, such that the second electrode 220 is spaced apart from the third electrode 230. The first electrodes 210 and the second electrode 220 are electrically connected together and are connected to a high-voltage electrode of a power supply (not shown) through a first conductive member 201, such as a conductive wire or other conductive members. The high-voltage electrode has a negative high voltage, which may range from -2 kV to -15 kV. The third electrode 230 is connected to a ground electrode of the power supply through a second conductive member 202, such as a ground wire or other conductive members. The first electrodes 210 are high-voltage negative ion electrodes 240, the second electrode 220 is a plasma high-voltage electrode 250, and the third electrode 230 is a plasma ground electrode 260. The negative ion electrode 240 forms monopolar tip corona discharge to generate negative ions. A voltage difference is formed between the plasma high-voltage electrode 250 and the plasma ground electrode 260 to generate plasmas. It can be understood that the protective cover 110 and the base 120 are made of an insulating material, such as plastic, ceramic, etc. The protective cover 110 prevents a person from touching the plurality of electrodes with hand, and can also provide physical and electrical protection for the plurality of electrodes, thereby achieving higher protection performance and higher safety.

Referring to FIG. 1, it can be understood that the first electrodes 210 and the second electrode 220 may extend into the base 120. For example, the base 120 may be provided with a groove having an opening facing toward the chamber formed by the protective cover 110 and the base 120, and the first electrodes 210 and the second electrode 220 are placed in the groove. As such, positioning and assembly of the first electrodes 210 and the second electrode 220 are facilitated, and the size can be reduced. The first conductive member 201 is connected to the first electrodes 210 and/or the second electrode 220 in the base 120, for example, in the groove, and extends out from the base 120 to connect to the high-voltage electrode of the power supply. The second conductive member 202 is connected to the third electrode 230 in the protective cover 110 and extends toward the base 120. The second conductive member 202 extends into the base 120 and extends out from the base 120 to connect to the ground electrode of the power supply. The first conductive member 201 and the second conductive member 202 may extend out from the same outlet hole in the base. The above wiring method can protect the first conductive member 201 and the second conductive member 202, avoid messy wiring, and reduce the space occupied by wires.

Referring to FIG. 1, it can be understood that two functional areas are formed in the protective cover 110, comprising a first functional area and a second functional area located in the chamber. The first functional area is a negative ion generating area 101 of the first electrode 210. In this embodiment, two first electrodes 210 are provided, i.e., two negative ion electrodes 240 are comprised, and the two negative ion electrodes 240 each form a negative ion generating area 101. However, the number of the first electrodes 210 is not limited to two. In some other embodiments, the number of the first electrodes 210 may be one or three or more. The second functional area is a plasma generating area 102 formed between the second electrode 220 and the third electrode 230. The negative ion generating areas are located on two sides of the plasma generating area 102. After the first electrodes 210 and the second electrode 220 are energized, negative ions are generated in the negative ion generating areas, and plasmas are generated in the plasma generating area 102. To reduce the impact of mutual interference between the negative ion generating areas and the plasma generating area 102 on the purification effect, a combined structure formed by the first electrodes 210, the second electrode 220, the third electrode 230, and the protective cover 110 is optimized, to facilitate the miniaturization design of the air purification device 1000. Details will be described in the following embodiments.

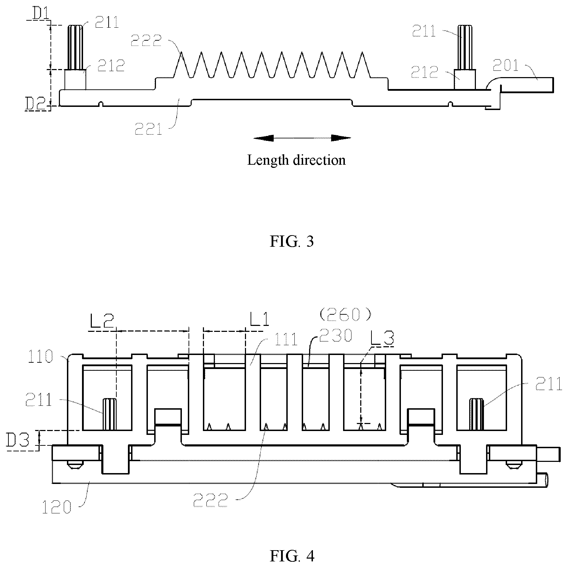

Referring to FIG. 2 and FIG. 3, the first electrodes 210 and the second electrode 220 is in a connected configuration. The second electrode 220 comprises an electrode plate 221. The two negative ion electrodes 240 are respectively connected to two ends of the electrode plate 221. In this embodiment, firstly, the negative ion electrodes 240 are fixed to the electrode plate 221 by welding, wires or other means, which is not limited herein. Then, the negative ion electrodes 240 and the electrode plate 221 are fixed to the base 120, and are connected to the high-voltage electrode of the same power supply through a conductive wire. As such, the negative ion electrodes 240 and the plasma high-voltage electrode 250 are powered and supplied with the same high-voltage through the conductive wire at the same time, and there is no need to provide two sets of power supplies. Therefore, the requirement of the ion generation assembly 200 for a high-voltage power supply is lowered, thereby reducing the costs.

Referring to FIG. 2 and FIG. 3, it can be understood that each of the negative ion electrodes 240 comprises a first tip portion 211, the electrode plate 221 comprises a second tip portion 222, and the second tip portion 222 is arranged facing toward the third electrode 230. In an embodiment of the present disclosure, the first tip portion 211 is made of a carbon fiber material and comprises a plurality of carbon fiber bristles, and the bottom of the negative ion electrode 240 is a basal portion 212. The carbon fiber bristles are pinched by the basal portion 212, root portions of the carbon fiber bristles are fixed to the basal portion 212, and the carbon fiber bristles extend out from the basal portion 212 to form a tip structure. After a negative high voltage is applied to the first tip portion 211, monopolar tip corona discharge is formed to generate negative ions. The carbon fiber bristles are welded to the electrode plate 221 through the basal portion 212 and are electrically connected to the electrode plate 221. As such, processing and assembly are simplified, and no wiring is needed, such that the space occupied can be reduced and miniaturization can be achieved. In some other embodiments, the first tip portion 211 may also be made of other conductive fibers, and the first tip portion 211 may also be a tooth-like structure, a needle tip structure, or other tip structures, which is not limited herein. For example, the first tip portion 211 may be a metal needle structure.

Referring to FIG. 1 and FIG. 3, it should be noted that the negative ion electrodes 240 are respectively arranged on the two sides of the plasma high-voltage electrode 250, the two first tip portions 211 are respectively located on two sides of the second tip portion 222, and the two first tip portions 211 are spaced apart from the second tip portion 222 by a certain distance. As such, the interference of the high-voltage discharge of the first tip portions 211 on the electric field of the plasma generating area 102 can be reduced, the local electric field can be made more uniform, and the distribution and diffusion of negative ions can be facilitated. In some embodiments, three or more negative ion electrodes 240 may be provided. The negative ion electrodes 240 are not limited to being arranged on the two sides of the electrode plate 221, and the negative ion electrodes 240 may also be arranged on the same side of the electrode plate 221, such that the negative ion generating areas 101 are located on one side of the plasma generating area 102.

Referring to FIG. 2 and FIG. 3, in an embodiment of the present disclosure, the electrode plate 221 is a metal plate, the third electrode 230 is a metal bar, the electrode plate 221 comprises a plurality of tooth-like second tip portions 222, and the plurality of second tip portions 222 are arranged in a length direction of the electrode plate 221 to form a sawtooth structure. The electrode plate 221 discharges through the sawtooth structure, and cooperates with the metal bar to generate a strong electric field to generate plasmas. It should be noted that the second tip portions 222 and the electrode plate 221 are integrally formed, and both the electrode plate 221 and the metal bar are made of stainless steel, to provide satisfactory corrosion resistance and improve the durability of the electrode structure. The electrode plate 221 and the metal bar may also be made of other corrosion-resistant materials. The second tip portions 222 may have a shape with a large curvature radius to provide strong discharge performance, such as a needle tip or a filament, which is not limited herein.

Referring to FIG. 1, FIG. 4, and FIG. 6, the protective cover 110 comprises a frame 112 and a plurality of grid bars 111 connected to the frame 112. The plurality of grid bars 111 are arranged at an interval in a length direction of the base 120 to form a grille, thereby constructing the hollowed-out structure. The frame 112 is generally rectangular. Each grid bar 111 is bent in an approximately U shape. Two ends of each of the grid bars 111 are respectively connected to the frame 112. The grid bars 111 and the frame 112 are integrally formed, to achieve a stable and reliable structure. A spacing is formed between adjacent grid bars 111. The negative ion generating areas 101 and the plasma generating area 102 are both in communicated with the spacing. The negative ions generated by the negative ion electrodes 240 can diffuse outward through the spacing, and air can also enter the plasma generating area 102 through the spacing. It should be noted that the spacing may be set to have different dimensions at different positions, or may be set to have a uniform dimension. As shown in FIG. 4, the spacing is defined as L1, and L1 ≤ 6 mm, i.e., a spacing between adjacent grid bars 111 is less than or equal to 6 mm, and the spacing is not zero. For example, the spacing may be set to 3 mm, 5 mm, etc., to ensure that a person cannot touch the electrodes in the protective cover 110 with a finger.

Referring to FIG. 4, it should be noted that the frame 112 is provided with a first buckle 121, and the base 120 is provided with a slot corresponding to the first buckle 121, and the first buckle 121 is buckled into the slot, thus connecting the protective cover 110 to the base 120. As such, the assembly is facilitated. In some other embodiments, a buckle may be provided on the base and a slot may be provided on the frame, and the assembly is realized by the engagement of the buckle with the slot. After the assembly of the protective cover 110 and the base 120 is completed, the two first tip portions 211 are both exposed out of the spacing in a width direction of the base 120, i.e., the grid bars 111 do not block the first tip portions 211, which is beneficial to speeding up the diffusion of negative ions, thereby improving the purification efficiency. It can be understood that after the air purification device 1000 is mounted in an air conditioner, a length direction of the protective cover 110 is perpendicular to or form an angle with the flow direction of air, such that an air flow can enter the protective cover 110 from the spacing on the side of the protective cover 110 in the width direction and directly blow toward the first tip portion 211. When the spacing satisfies the above conditions, the distance of the spacing near the first electrode 210 may be appropriately increased, or the distance of the spacing corresponding to the first tip portion 211 may be set to be larger than the distance of the spacing at other positions, to further improve the diffusion efficiency of negative ions.

Referring to FIG. 4, a height of the first tip portion 211 is greater than a height of the basal portion 212, and a sum of the height of the first tip portion 211 and the height of the basal portion 212 is greater than a height of the frame 112, such that the first tip portion 211 is not completely blocked by the frame 112, i.e., it is ensured that at least part of the first tip portion is higher than the frame 112 and can be exposed out of the spacing, so as not to affect the diffusion of negative ions. In an embodiment, the height of the basal portion 212 is set to be greater than the height of the frame 112, such that the first tip portion 211 can be completely exposed out of the spacing.

Referring to FIG. 1, FIG. 4, and FIG. 5, the protective cover 110 is further provided with a plurality of connecting ribs 113 connected to the grid bars 111, and the connecting ribs 113 are arranged in the length direction of the protective cover 110. The connecting ribs 113 can improve the stability of the grille structure, making the entire protective cover 110 more solid and more reliable. The metal bar is fixed to at least one connecting rib 113 on a top wall of the protective cover 110. The metal bar is located on a bottom surface of the connecting rib 113. The connecting rib 113 covers the metal bar to protect the metal bar, so as to prevent a human hand or other objects from accessing the metal bar from the outside of the protective cover 110, thereby ensuring electrical safety. It can be understood that the plasma ground electrode 260 is fixed to the protective cover 110, to save the space between the protective cover 110 and the plasma high-voltage electrode 250, which facilitates the miniaturization design of the overall structure, and lowers the requirement of the air purification device 1000 for installation space.

Referring to FIG. 1 and FIG. 4, it should be noted that the plasma high-voltage electrode 250 is fixed to the base 120, and the plasma ground electrode 260 is fixed to the protective cover 110. By adjusting the height of the protective cover 110, the distance between the metal bar and the sawtooth structure can be adjusted to achieve the purpose of adjusting the plasma intensity and ensuring electrical safety. The intensity of the plasmas generated may also be changed by adjusting the supply voltage. The matching relationship between the distance between the two electrodes and the supplied high-voltage electricity is adjusted according to an actual application scenario to achieve a satisfactory purification effect.

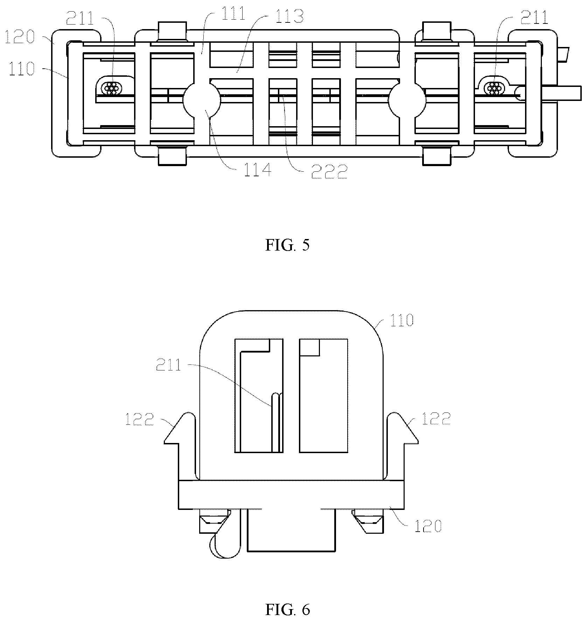

Referring to FIG. 5, FIG. 5 is a top view of the air purification device 1000. It should be noted that in a height direction of the protective cover 110, the metal plate and the metal bar are staggered and not aligned, i.e., the metal bar is not located exactly above the metal plate, and the direction of the electric field formed between the metal plate and the metal bar has an inclined angle. As such, the size of the air purification device 1000 is further reduced while ensuring the required distance between the metal plate and the metal bar. Moreover, in an embodiment, a length of the metal bar is greater than a length of a discharge portion of the metal plate, for example, a length of the sawtooth structure. As such, a fan-shaped plasma generating area 102 can be formed on the premise of ensuring a sufficient safety distance between the two electrodes, which is beneficial to increasing the spatial range of the plasma generating area 102 and improving the purification effect.

Referring to FIG. 5, it can be understood that in an embodiment, a length by which the metal bar extends in the length direction of the base 120 is less than or equal to a length of the connecting rib 113, and a width of the metal bar is less than or equal to a width of the connecting rib 113, i.e., the connecting rib 113 can completely cover the metal bar, and a person cannot touch the metal bar with a finger from the outside of the protective cover 110. It should be noted that connecting lugs (not shown) are respectively arranged at two ends of the metal bar, shielding portions 114 matching the connecting lugs are respectively arranged on the two grid bars 111, and the two connecting lugs are respectively fixedly connected to bottom surfaces of the two shielding portions 114, such that the connecting lugs can be shielded by the shielding portions 114, thereby further improving electrical safety. In addition, the metal bar is connected to the ground electrode of the power supply through a conductive wire. The conductive wire extends from the top wall of the protective cover 110 to the base 120 and is led out from the base 120 to connect to the power supply.

Referring to FIG. 4, in an embodiment, a distance between the first tip portion 211 and the metal bar is defined as L2, the distance L2 is a spacing between the first tip portion 211 and the metal bar in the length direction, a distance between the second tip portion 222 and the metal bar is defined as L3, and L2 > L3, i.e., a spacing between the first tip portion 211 and the metal bar needs to be greater than a spacing between the second tip portion 222 and the metal bar, such that the interference of the negative ion generating area 101 to the plasma generating area 102 can be reduced. It can be understood that both the first tip portion 211 and the second tip portion 222 are high-voltage electrodes, and if the first tip portion 211 is closer to the metal bar than the second tip portion 222 is, the electric field formed between the second tip portion 222 and the metal bar will be interfered, and the formation of the plasma generating area 102 will be affected, affecting the generation of plasmas. When the condition L2 > L3 is satisfied, the first tip portion 211 is spaced apart from the metal bar by a sufficiently large distance to achieve an electrode discharge effect and generate negative ions, and the second tip portion 222 can cooperate with the metal bar to generate a stable electric field, thereby ensuring the stability of the plasma generating area 102.

Referring to FIG. 4 and FIG. 5, in an embodiment, considering that the negative ion electrodes 240 and the plasma high-voltage electrode 250 are connected to a high voltage during operation, a distance between the negative ion electrode 240 and the protective cover 110 is set to be greater than or equal to 3 mm, and a distance between the plasma high-voltage electrode 250 and the protective cover 110 is also set to be greater than or equal to 3 mm. It can be understood that both the negative ion electrodes 240 and the plasma high-voltage electrode 250 are connected to the base 120. Therefore, the distance between the negative ion electrode 240 and the protective cover 110 in each direction should be not less than 3 mm, and the distance between the plasma high-voltage electrode 250 and the protective cover 110 in each direction should be not less than 3 mm. For example, the negative ion electrode 240 is spaced apart from the protective cover 110 by 3 mm, 4 mm, 6 mm, etc.

Referring to FIG. 6, FIG. 6 is a schematic side view of the air purification device 1000. The base 120 is further provided with a second buckle 122 configured for connecting to an indoor unit of an air conditioner. As such, the air purification device 1000 can be conveniently and quickly assembled to the indoor unit of the air conditioner. Alternatively, a buckle may be provided on the frame 112 to connect to the indoor unit of the air conditioner. The method for mounting the air purification device 1000 on the indoor unit of the air conditioner is not limited to buckling, and bolt connection or other methods may also be used, which is not limited herein.

An embodiment of the present disclosure provides an air conditioner (not shown), comprising the air purification device 1000 of the above embodiments. Using an indoor unit of the air conditioner as an example, the air purification device 1000 may be mounted at an air outlet of the indoor unit of the air conditioner. An air flow generated at the air outlet is beneficial to speeding up the diffusion of negative ions. The air conditioner has the advantages of negative ions and plasmas, can actively diffuse negative ions into the air to achieve a sterilization effect, can kill bacteria and viruses in the air passing through the air purification device 1000, and also provides a certain odor removal effect, such that indoor air can be purified to improve indoor air quality.

The embodiments of the present disclosure have been described in detail above in conjunction with the accompanying drawings, but the present disclosure is not limited to the above embodiments, and various changes may be made within the knowledge of those having ordinary skills in the art without departing from the scope of the present disclosure.

## Claims

1. An air purification device comprising:
a housing comprising a protective cover and a base, wherein the base is connected to the protective cover; and
an ion generation assembly comprising at least one first electrode, a second electrode, and a third electrode, wherein the at least one first electrode is configured for discharging to generate negative ions, and the second electrode is spaced apart from the third electrode and is configured for cooperating with the third electrode to generate plasmas,
wherein the protective cover is of a hollowed-out structure, the hollowed-out structure covers the ion generation assembly, the at least one first electrode and the second electrode are connected to the base, and the third electrode is connected to an inner side of the protective cover.

2. The air purification device of claim 1, wherein the at least one first electrode and the second electrode are electrically connected, and are connected to a high-voltage electrode of a power supply through a first conductive member, and the third electrode is connected to a ground electrode of the power supply through a second conductive member.

3. The air purification device of claim 2, wherein the at least one first electrode and the second electrode are fixedly connected to form a unity or are connected by a wire.

4. The air purification device of claim 2, wherein:
the at least one first electrode and the second electrode extend into the base, and the first conductive member is connected to the at least one first electrode or the second electrode in the base and extends out from the base to connect to the high-voltage electrode of the power supply; and
the second conductive member is connected to the third electrode in the protective cover and extends towards the base, and the second conductive member extends into the base and extends out from the base to connect to the ground electrode of the power supply.

5. The air purification device of any one of claims 1 to 4, wherein the second electrode comprises an electrode plate, the electrode plate comprises a plurality of second tip portions arranged in a length direction of the electrode plate, and the at least one first electrode is connected to an end of the electrode plate.

6. The air purification device of claim 5, wherein the at least one first electrode comprises at least two first electrodes, and the at least two first electrodes are divided into two groups and respectively arranged at two ends of the electrode plate, or the at least two first electrodes are both arranged at an end of the electrode plate.

7. The air purification device of claim 5, wherein the second tip portion is of a tooth-like, needle-tip, or filament-like structure.

8. The air purification device of any one of claims 1 to 4, wherein a connecting rib is arranged on a side wall of the protective cover away from the base, the third electrode is arranged on a side of the connecting rib facing the base, and the connecting rib, the second electrode, and the third electrode respectively extend in a length direction of the base.

9. The air purification device of claim 8, wherein a length by which the third electrode extends in the length direction of the base is less than or equal to a length of the connecting rib, and a width of the third electrode is less than or equal to a width of the connecting rib.

10. The air purification device of claim 8, wherein shielding portions are arranged on the side wall of the protective cover away from the base, the shielding portions are respectively located at two ends of the connecting rib, connecting portions are respectively arranged at two ends of the third electrode, and the connecting portions are connected to a side of the shielding portions facing the base.

11. The air purification device of claim 8, wherein a length of the third electrode is greater than a length of a discharge portion of the second electrode.

12. The air purification device of claim 8, wherein the third electrode and the second electrode are staggered in a height direction of the base.

13. The air purification device of any one of claims 1 to 4, wherein the protective cover comprises a frame and a plurality of grid bars connected to the frame, the plurality of grid bars are arranged at an interval in a length direction of the base to form a grille, thereby constructing the hollowed-out structure, a spacing is formed between adjacent grid bars, and a discharge portion of the at least one first electrode and/or a discharge portion of the second electrode is exposed through the spacing in a width direction of the base.

14. The air purification device of claim 13, wherein the spacing between adjacent grid bars is less than or equal to 6 mm.

15. The air purification device of any one of claims 1 to 4, wherein the at least one first electrode comprises a basal portion and a first tip portion connected to a top end of the basal portion, and a height of the first tip portion is greater than a height of the basal portion.

16. The air purification device of claim 15, wherein the first tip portion comprises a plurality of conductive fibers connected to the basal portion.

17. The air purification device of any one of claims 1 to 4, wherein a distance between the at least one first electrode and the third electrode is greater than a distance between the second electrode and the third electrode.

18. The air purification device of any one of claims 1 to 4, wherein a distance between the at least one first electrode and the protective cover is greater than or equal to 3 mm, and a distance between the second electrode and the protective cover is greater than or equal to 3 mm.

19. An air conditioner, comprising an air purification device of any one of claims 1 to 18.
